# EUROPÄISCHE PATENTSCHRIFT

(11) **EP 2 555 806 B1**
(45) Veröffentlichungstag und Bekanntmachung des Hinweises auf die Patenterteilung: **08.05.2019**
(21) Anmeldenummer: 11727907.5
(22) Anmeldetag: 29.03.2011
(51) Int. Cl.: A61L 24/06, A61L 26/00, C08L 33/12, A61L 15/58

(54) **ACRYLATKLEBSTOFF FÜR ANWENDUNGEN AUF DER HAUT**
ACRYLATE ADHESIVE FOR USE ON THE SKIN
ADHÉSIF À BASE D'ACRYLATE POUR APPLICATIONS CUTANÉES

(30) Priorität: 03.04.2010 DE 102010013799
(43) Veröffentlichungstag der Anmeldung: 13.02.2013
(73) Patentinhaber: Lohmann GmbH & Co. KG, 56567 Neuwied (DE)
(72) Erfinder: KLUGE, Thomas, 56179 Vallender (DE); NISSING, Peter, 56317 Urbach (DE); PETRICK, Patricia, 56355 Oberbachheim (DE); SCHÜTTE, Mario, 56072 Koblenz (DE)
(74) Vertreter: Schweitzer, Klaus
(86) Internationale Anmeldenummer: PCT/DE2011/000344
(87) Internationale Veröffentlichungsnummer: WO 2011/120507

(56) Entgegenhaltungen:
- EP-A2- 1 357 140
- EP-A2- 1 357 140
- WO-A1-91/14461
- WO-A1-91/14461
- WO-A1-98/03208

## Beschreibung

Die vorliegende Erfindung betrifft die Verwendung einer Haftklebemasse zur Verklebung auf der menschlichen Haut. Die erfindungsgemäß eingesetzten Monomerbestandteile der polymeren Haftklebemasse bestehen dabei zu 100% aus acrylat-basierten Monomeren. Die daraus hergestellten Klebefilme in einer Schichtdicke von ca. 250 µm weisen eine sichere Haftung auf der menschlichen Haut auf, besitzen trotz der Schichtdicke eine hohe Wasserdampfdurchlässigkeit, wodurch z.B. die Wundheilung begünstigt wird und sind auch nach längerer Tragezeit rückstandsfrei und ohne Schädigung der Haut von dieser wieder ablösbar.

Eine Vielzahl von Anwendungen im Medikalbereich erfordert die Befestigung auf der menschlichen Haut mittels geeigneter Haftklebstoffe. Auf Grund der Sensibilität dieser Anwendungen kommen hier spezielle Haftklebstoffe zum Einsatz. Diese müssen die an sich gegensätzlichen Anforderungen von zum einen einer sicheren Verklebung auf der Haut in allen ihren in Abhängigkeit von Alter und Gesundheitszustand des Patienten variierenden Varianten während der Anwendung und zum anderen einer möglichst schmerz- und rückstandsfreie Ablösung nach der Anwendung erfüllen. Darüber hinaus dürfen sie keine sensibilisierenden oder in anderer Hinsicht hautschädigenden Stoffe enthalten. Haftklebstoffe auf Acrylatbasis stellen eine in diesem Bereich häufig verwendete Klasse von Klebstoffen dar. Der Vorteil dieser Materialien liegt in ihrer hervorragenden Haftung, der guten Verträglichkeit der Inhaltsstoffe zur menschlichen Haut, der geringen Neigung zur Sensibilisierung und der sehr guten Sterilisations- und Alterungsbeständigkeit.

So beschreibt EP 0 099 748 B1 einen Wundverband basierend auf einem Polyacrylat-Haftklebstoff mit hoher Wasserdampfdurchlässigkeit. Nachteilig ist die Neigung der acrylatbasierenden Haftklebstoffe zum Anstieg der Verbundhaftung mit zunehmender Tragezeit bedingt durch eine zunehmende Verbesserung der Benetzung der Haut durch den Haftklebstoff. Dies ist insbesondere dann nachteilig, wenn das haftklebende Produkt aus therapeutischen Gesichtspunkten lange Zeit auf der Haut verbleiben und danach von dieser wieder mechanisch entfernt werden muss. In diesem Fall kommt es in der Praxis häufig zu Verletzungen der Haut durch Heraustrennen von Hautzellen. Um diesem Effekt zu begegnen, werden hochmolekulare Acrylathaftklebstoffe in Verbindung mit weichmachenden Additiven als Klebstoffe für Wundverbände eingesetzt. Ein solche Lösung wird beispielsweise in den Patentschriften EP 0 891 782 B1 und EP 0 435 199 B1 beschrieben. Der Einsatz der weichmachenden Inhaltsstoffe bewirkt in Zusammenhang mit der hochmolekularen Polymermatrix eine sichere Haftung auf der menschlichen Haut. Nachteilig wirkt sich hier jedoch die Migrationsneigung der weichmachenden Komponenten aus. Dies bedingt als Folge der Mischung zweier Komponenten mit unterschiedlichem Haftverhalten ein uneinheitliches Abziehverhalten von der Haut.

Aus diesem Grund setzt man für derartige Anwendungen nach dem aktuellen Stand der Technik Silikon-Gel Klebstoffe ein. Diese haben den Vorteil, dass sie auch nach langer Tragezeit leicht und ohne Hautschädigung von der Haut abgezogen werden können. So beschreibt US 4,921,704 einen Wundverband für exsudierende Wunden, bestehend aus einer porösen Klebeschicht auf Basis eine Silikon-Gel-Klebstoffes und einer äußeren Absorptionsschicht. US 5,635,201 beschreibt die Herstellung eines Wundverbandes durch Applikation einer Silikon-Gel-Haftklebstoffschicht auf ein poröses Substrat unter Erhalt der Porösität. Nachteilig sind bei diesen Lösungen neben den erhöhten Kosten die gegenüber acrylatbasierenden Haftklebstoffen geringere Klebkraft und die sehr geringe Wasserdampfdurchlässigkeit. Diese hat zur Folge, dass Haftklebeprodukte auf Basis von Silikon-Gel-Klebstoffen auf der menschlichen Haut nicht vollflächig appliziert werden können, da anderenfalls durch die fehlende Atmungsaktivität dieser Klebefilme Mazerationen der Haut vor allem bei längeren Tragezeiten auftreten können. Die Herstellung der unterbrochenen Klebstoffschichten mit diesen Haftklebstoffen ist technologisch sehr aufwändig und bewirkt eine weitere Verschlechterung der Klebkraft im Vergleich zu acrylatbasierenden Haftklebstoffen.

Die WO 91/14461 A offenbart Acrylat-basierte Haftklebemassen für ölige Haut.

Vor diesem Hintergrund war es Aufgabe der vorliegenden Erfindung, Haftklebstoffe auf Acrylatbasis bereitzustellen, die eine gute Klebkraft auf der menschlichen Haut aufweisen, eine hohe Wasserdampfdurchlässigkeit auch bei höheren Schichtdicken besitzen, eine gute Bioverträglichkeit zeigen und auch nach längerer Tragezeit von der menschlichen Haut ohne Schädigung abgezogen werden können.

Diese Aufgabe konnte in überraschender und für den Fachmann nicht vorhersehbarer Weise durch die erfindungsgemäßen Haftklebstoffe gelöst werden.

Die erfindungsgemäßen Haftklebstoffe können dabei durch Polymerisation der Monomere in Gegenwart eines radikalischen Initiators hergestellt werden. Dies kann sowohl in einem geeigneten Lösemittel als auch lösemittelfrei erfolgen. Die lösemittelfreie Ausführungsform ist hierbei besonders bevorzugt.

Als Monomere zur Herstellung der erfindungsgemäßen Haftklebstoffe können ethylenisch ungesättigte Verbindungen wie z.B. (Meth)acrylate eingesetzt werden. (Meth)acrylate im Sinne der vorliegenden Erfindung sind Veresterungsprodukte von Acrylsäure oder Methacrylsäure mit einwertigen Alkoholen. Vorzugsweise sind die (Meth)acrylate Veresterungsprodukte von Acrylsäure oder Meth(acrylsäure) mit einwertigen oder mehrwertigen 1 bis 20 C-Atome, bevorzugt 6 bis 10 C-Atome enthaltenden Alkoholen, wobei die Alkoholfunktion als primärer, sekundärer, tertiärer oder cyclischer Rest vorliegen kann. Insbesondere sind die Meth(Acrylate) aus der Gruppe ausgewählt, die aus Methyl(meth)acrylat, Ethyl(meth)acrylat, Butyl (meth)acrylat, Hexyl(meth)acrylat, Isooctyl-(meth)acrylat, 2-Ethylhexyl(meth)acrylat, Isodecyl(meth)acrylat, Isobornyl(meth)acrylat, Ethoxy(ethoxy)ethyl(meth)acrylat, Phenoxyethylacrylat, Hexandiolacrylat, Hexandioldiacrylat, Hydroxyalkyl(meth)acrylat mit einem Alkylrest von vorzugsweise 1-20 C-Atomen, Polyethylenglycol(meth)acrylat mit 5-10 Ethylenoxid-Einheiten, (Meth) acrylsäure und Glycidyl(meth) acrylat besteht.

Ebenso können Mischungen der zuvor genannten Verbindungen eingesetzt werden.

Als Initiatoren für die radikalische Polymerisation der Monomere können sowohl wasser- als auch öllösliche Verbindungen verwendet werden, die nach thermischer, chemischer oder elektromagnetischer Aktivierung reaktionsfähige Radikale bilden, insbesondere Peroxodisulfate und organische Hydroperoxide, wie Kaliumperoxodisulfat, Natriumperoxodisulfat, Cumolhydroperoxid, Azobisisobutyronitril, Butylhydroperoxid, m-Chlorperbenzoesäure, Benzophenon, Bis(2,6-dimethoxybenzoyl)-2,4,4-trimethyl-pentylphosphinoxid, 2,4,6-Trimethylbenzophenon, 2-Hydroxy-2-methyl-1-phenyl-1-propanon, 2,4,6 Trimethylbenzoyldiphenylposhinoxid, 1-[4-(2-Hydroxy-ethoxy)-phenyl]-2-hydroxy-2-methyl-l-propanon und 1-Hydroxy-cyclohexyl-phenyl-keton, sowie Mischungen dieser Initiatoren.

Zur Polymerisation der Monomerkomponenten können sowohl anorganische als auch organische Reduktionsmittel verwendet werden, vorzugsweise Alkalimetallsalze der schwefligen oder dischwefligen Säure wie Natriumsulfit, Natriumdisulfit oder Natriumhydrogensulfit, Hydroxymethansulfinsäure und deren Salze sowie primäre und sekundäre Amine, wie Triethylamin und Diethylentetramin und Mischungen der zuvor genannten Verbindungen.

Die Initiatoren und Reduktionsmittel werden bevorzugt in einem Anteil von 0,1 - 5 Gew.-%, besonders bevorzugt in einem Anteil von 0,5 - 3 Gew.-% bezogen auf die Menge der Monomerkomponenten eingesetzt.

Die Polymerisation der erfindungsgemäßen Haftklebstoffe kann sowohl durch Temperatur als auch durch aktinische Strahlung ausgelöst werden. Besonders bevorzugt ist die Initiierung der Polymerisationsreaktion durch UV-Strahlung.

Die Polymerisation der erfindungsgemäßen Haftklebstoffe kann entweder in einem lösemittelbasierenden, wasserbasierenden oder lösemittelfreien Verfahren durchgeführt werden. Diese Verfahren sind dem Fachmann bekannt.

Die vorliegende Erfindung ist ferner auf die Verwendung der erfindungsgemäßen Polymere zur Verklebung auf der menschlichen Haut gerichtet, z.B. im Bereich der Wundversorgung, der transdermalen therapeutischen Systeme oder der Ostomieversorgung.

Zur Einstellung der viskoelastischen Eigenschaften kann die Zugabe mehrfunktioneller Monomere zum Monomergemisch erfolgen. Solche mehrfunktionellen Verbindungen sind beispielsweise Tripropylenglycoldiacrylat, Trimethylolpropantriacrylat und Pentaerythrol-(meth)acrylat. Der Vernetzeranteil in den erfindungsgemäßen Haftklebemassen beträgt 0,01 % bis 5,0 %, bevorzugt von 0,07 % bis 3,0 % und besonders bevorzugt von 0,15 % bis 1,8 %.

Als Abdeckmaterialien der Klebmasse können die dem Fachmann bekannten Flächenmaterialien eingesetzt werden, die gegenüber den erfindungsgemäßen Haftklebstoffen ein Release-Niveau aufweisen, welches eine sichere Lagerung und Verarbeitung in Rollen- und Stanzteilform ermöglicht. Hierzu gehören beispielsweise silikonisierte Folien auf Basis Polyester, Polyethylen und Polypropylen sowie silikonisierte Papiere.

Die nachfolgenden Beispiele verdeutlichen die vorliegende Erfindung, ohne diese auf die Ausführungsbeispiele zu beschränken.

### Beispiele 1 - 5 Herstellung der erfindungsgemäßen Polymere und der Klebefilme

Für jedes der Beispiele 1 - 5 wird zunächst in einem Dreihalskolben mit Rührer, Rückflußkühler und Tropftrichter ein Monomerengemisch, bestehend aus 50 GT Polyethylenglykolmethacrylat 550 und 50 GT 2-Ethylhexylacrylat zusammen mit 3 GT des Initiators 2,4,6-Trimethylbenzophenon vorgelegt. Danach wird die Mischung über einen Zeitraum von 10 min mit einer UV-Lampe bestrahlt. Anschließend wird die Mischung über einen 50µm Filter ausgetragen. Der Umsatz beträgt 36%.

### Beispiel 1

Zu 800GT des vorab hergestellten und beschriebenen Polymerisats werden 6GT Tripropylenglykoldiacrylat und 10GT des Initiators 2,4,6-Trimethylbenzophenon gegeben. Die Mischung wird mittels eines Laborrakels mit einer Schichtdicke von 250µm beschichtet und mit einer UV-Lampe über einen Zeitraum von 30s bestrahlt. Der Restmonomerengehalt der so erhaltenen Klebefilme beträgt 120ppm.

### Beispiel 2

Zu 800GT des vorab hergestellten und beschriebenen Polymerisats werden 4GT Tripropylenglykoldiacrylat und 10GT des Initiators 2,4,6-Trimethylbenzophenon gegeben. Die Mischung wird mittels eines Laborrakels mit einer Schichtdicke von 250µm beschichtet und mit einer UV-Lampe über einen Zeitraum von 30s bestrahlt. Der Restmonomerengehalt der so erhaltenen Klebefilme beträgt 150ppm.

### Beispiel 3

Zu 800GT des vorab hergestellten und beschriebenen Polymerisats werden 2GT Tripropylenglykoldiacrylat und 10GT des Initiators 2,4,6-Trimethylbenzophenon gegeben. Die Mischung wird mittels eines Laborrakels mit einer Schichtdicke von 250µm beschichtet und mit einer UV-Lampe über einen Zeitraum von 30s bestrahlt. Der Restmonomerengehalt der so erhaltenen Klebefilme beträgt 170ppm.

### Beispiel 4 (nicht Bestandteil der Ansprüche)

Zu 800GT des vorab hergestellten und beschriebenen Polymerisats werden 2GT Tripropylenglykoldiacrylat und 10GT des Initiators Azobisisobutyronitril gegeben. Die Mischung wird mittels eines Laborrakels mit einer Schichtdicke von 250µm beschichtet und mit einer NIR-Lampe ("Lambda Technologies") über einen Zeitraum von 90s bestrahlt. Der Restmonomerengehalt der so erhaltenen Klebefilme beträgt 200ppm.

### Beispiel 5

In einem Dreihalskolben mit Rührer, Rückflußkühler und Tropftrichter werden 460 GT Ethylacetat vorgelegt und unter Rühren bis zum Sieden erhitzt. Nach Erreichen des Siedepunktes werden 30% eines Monomerengemisches, bestehend aus 2GT Acrylsäure, 25GT Ethylhexylacrylat und 25GT Polyethylenglykolmethacrylat und 30% der Starterlösung bestehend aus 0,3 GT Azobisisobutyronitril in 50 GT Ethylacetat vorgelegt. Danach werden parallel die restlichen 70% des Monomerengemisches und der Starterlösung über einen Zeitraum von 60 min zudosiert. Anschließend wird nochmals eine Lösung von 0,1 GT Azobisisobutyronitril in 90 GT Ethylacetat zugegeben und für 120 min gerührt, abgekühlt und über einen 50µm Filter ausgetragen. Das Polymerisat weist ein mittleres Molekulargewicht von 780000 auf. Die Polydispersität beträgt 10,1. Der Restmonomerengehalt beträgt 200ppm. Das Produkt wird mittels eines Laborrakels mit einer Schichtdicke von 125µm beschichtet. Zum Erreichen der Endschichtdicke von 250µm werden zwei dieser Filme bei Raumtemperatur mit einem Druck von 3 bar zusammenkaschiert.

### Klebefilmprüfung

Die Wasserdampfdurchlässigkeit wurde zum einen in Anlehnung an DIN EN 13726-2 bei einer Temperatur von 37°C und einer Luftfeuchtigkeit von 18% bestimmt und zum anderen in Anlehnung an ASTM E 96 nach der "upright-cup"-Methode bei einer Temperatur von 23° C. und einer Luftfeuchtigkeit von 50%. Für die Bewertung des Low-Trauma-Verhaltens wurde ein Tragetest über einen Zeitraum von 24h durchgeführt, welcher nach folgenden qualitativen Kriterien bewertet wurde:
1- keine Kleberrückstände auf der Haut, keine Ablösung von Hautzellen beim Abziehen
2- keine Klebeberrückstände auf der Haut, geringe Ablösung von Hautzellen beim Abziehen (<5% der Klebefläche)
3- geringe Kleberrückstände (<5% der Klebefläche) auf der Haut, geringe Ablösung von Hautzellen beim Abziehen
4- geringe Kleberrückstände auf der Haut, starke Ablösung von Hautzellen beim Abziehen
5- starke Kleberückstände auf der Haut, starke Ablösung von Hautzellen beim Abziehen

Im Rahmen des Tragetestes wurde auch der sichere Halt auf der menschlichen Haut bewertet. Hierbei wurde der folgende Bewertungsmaßstab zugrunde gelegt:
1 - sehr gute Haftung, keine Ablösung unter Belastung
2 - gute Haftung, vereinzelte Ablösung an den Ecken
3 - schlechte Haftung, starke Ablösung an Ecken
4 - sehr schlechte Haftung, vereinzelte Komplettablösung
5 - keine Haftung, häufige Komplettablösung

Mit den in den angegebenen Beispielen hergestellten Klebemitteln ergaben sich in Anlehnung an ASTM E 96 nach der "upright-cup"-Methode bei einer Temperatur von 23° C. und einer Luftfeuchtigkeit von 50% folgende Ergebnisse:

| | Einheit | Beispiel 1 | Beispiel 2 | Beispiel 3 | Beispiel 4 | Beispiel 5 |
|---|---|---|---|---|---|---|
| Wasserdampfdurchlässigkeit | g/m² 24h | 1280 | 1390 | 1530 | 1330 | 1070 |
| Tragetest | 1-5 | 1 | 1-2 | 2 | 1-2 | 2 |
| Haftung auf der Haut | 1-5 | 1-2 | 1 | 1 | 1-2 | 2 |

In Anlehnung an DIN EN 13726-2 zeigten der Tragetest und die Haftung auf der Haut bei einer Temperatur von 37°C und einer Luftfeuchtigkeit von 18% ähnliche Ergebnisse, die Wasserdampfdurchlässigkeit lag hier in allen Fällen zwischen 1000 und 1200 g/m² über 24 h.

Die Werte zeigen, dass bei allen erfindungsgemäßen Klebefilmen sowohl eine hohe Wasserdampfdurchlässigkeit von >1000g/m² über 24h als auch keine oder nur eine minimale Schädigung der Epidermis erreicht wurde. Alle Muster zeigten im Tragetest eine sichere Haftung auf der menschlichen Haut.

## Patentansprüche

1. Verwendung einer polymeren Haftklebemasse, die zu 100% aus acrylat-basierten Monomeren resultiert und bei einer Schichtdicke von 250µm eine Wasserdampfdurchlässigkeit gemessen
- nach ASTM E 96 nach der "upright cup"-Methode bei einer Temperatur von 23 °C sowie einer Luftfeuchtigkeit von 50% und
- nach DIN EN 13726-2 bei einer Temperatur von 37 °C sowie einer Luftfeuchtigkeit von 18%
von mindestens 1.000 g/m² über 24h aufweist,
zur Verklebung auf der menschlichen Haut.

2. Verwendung nach Anspruch 1, **dadurch gekennzeichnet, dass** die Haftklebemasse durch UV-Polymerisation hergestellt wird.

3. Verwendung nach Anspruch 1, **dadurch gekennzeichnet, dass** die Haftklebemasse durch thermische Polymerisation hergestellt wird.

4. Verwendung nach Anspruch 1, **dadurch gekennzeichnet, dass** die Haftklebemasse durch Polymerisation ohne Verwendung von organischen Lösemitteln hergestellt wird.

5. Verwendung nach Anspruch 1, **dadurch gekennzeichnet, dass** die Haftklebemasse durch Polymerisation auf Lösemittelbasis hergestellt wird

6. Verwendung nach einem der vorhergehenden Ansprüche, **dadurch gekennzeichnet, dass** die Haftklebemasse auch nach einer Tragezeit von bis zu 24 h ohne Kleberrückstände und mit einer maximal auf 5% der Klebefläche beschränkten Ablösung von Hautzellen von der menschlichen Haut abgelöst werden kann.

7. Polymere Haftklebemasse zur Verklebung auf der menschlichen Haut, resultierend aus 100% acrylat-basierten Monomeren, nämlich
50 Gewichtsteilen Polyethylenglykolmethacrylat 550 und
50 Gewichtsteilen 2-Ethylhexylacrylat und
0.75 Gewichtsteile Tripropylenglykoldiacrylat,
wobei die Haftklebemasse bei einer Schichtdicke von 250µm eine Wasserdampfdurchlässigkeit gemessen
- nach ASTM E 96 nach der "upright cup"-Methode bei einer Temperatur von 23 °C sowie einer Luftfeuchtigkeit von 50% bei einer Bestrahlung mit einer UV-Lampe über einen Zeitraum von 30s von 1280 g/m²·24h, und
- nach DIN EN 13726-2 bei einer Temperatur von 37 °C sowie einer Luftfeuchtigkeit von 18% zwischen 1.000 und 1200 g/m² über 24h aufweist,
oder
50 Gewichtsteilen Polyethylenglykolmethacrylat 550 und
50 Gewichtsteilen 2-Ethylhexylacrylat und
0.5 Gewichtsteile Tripropylenglykoldiacrylat,
wobei die Haftklebemasse bei einer Schichtdicke von 250µm eine Wasserdampfdurchlässigkeit gemessen
- nach ASTM E 96 nach der "upright cup"-Methode bei einer Temperatur von 23 °C sowie einer Luftfeuchtigkeit von 50% bei einer Bestrahlung mit einer UV-Lampe über einen Zeitraum von 30s von 1390 g/m²·24h, und
- nach DIN EN 13726-2 bei einer Temperatur von 37 °C sowie einer Luftfeuchtigkeit von 18% zwischen 1.000 und 1200 g/m² über 24h aufweist
oder
50 Gewichtsteilen Polyethylenglykolmethacrylat 550 und
50 Gewichtsteilen 2-Ethylhexylacrylat und
0.25 Gewichtsteile Tripropylenglykoldiacrylat,
wobei die Haftklebemasse bei einer Schichtdicke von 250µm eine Wasserdampfdurchlässigkeit gemessen
- nach ASTM E 96 nach der "upright cup"-Methode bei einer Temperatur von 23 °C sowie einer Luftfeuchtigkeit von 50% bei einer Bestrahlung mit einer UV-Lampe über einen Zeitraum von 30s von 1530 g/m²·24h, und
- nach DIN EN 13726-2 bei einer Temperatur von 37 °C sowie einer Luftfeuchtigkeit von 18% zwischen 1.000 und 1200 g/m² über 24h aufweist
oder
2 Gewichtsteilen Acrylsäure und
25 Gewichtsteilen Ethylhexylacrylat und
25 Gewichtsteile Polyethylenglykolmethacrylat,
wobei die Haftklebemasse bei einer Schichtdicke von 250µm eine Wasserdampfdurchlässigkeit gemessen
- nach ASTM E 96 nach der "upright cup"-Methode bei einer Temperatur von 23 °C sowie einer Luftfeuchtigkeit von 50% von 1070 g/m²·24h, und
- nach DIN EN 13726-2 bei einer Temperatur von 37 °C sowie einer Luftfeuchtigkeit von 18% zwischen 1.000 und 1200 g/m² über 24h aufweist.

## Claims

1. Use of a polymeric, pressure-sensitive adhesive resulting 100 % from acrylate-based monomers and having at a layer thickness of 250 *µ*m a water vapour permeability, as measured
- to ASTM E 96 by the upright cup method at a temperature of 23 °C and an atmospheric humidity of 50 % and
- to DIN EN 13726-2 at a temperature of 37 °C and an atmospheric humidity of 18 %,
of at least 1000 g/m² over 24 h
for bonding to human skin.

2. Use according to Claim 1, **characterized in that** the pressure-sensitive adhesive is prepared by UV polymerization.

3. Use according to Claim 1, **characterized in that** the pressure-sensitive adhesive is prepared by thermal polymerization.

4. Use according to Claim 1, **characterized in that** the pressure-sensitive adhesive is prepared by polymerization without using organic solvents.

5. Use according to Claim 1, **characterized in that** the pressure-sensitive adhesive is prepared by solvent-based polymerization.

6. Use according to any of the preceding claims, **characterized in that** the pressure-sensitive adhesive, even after a wearing time of up to 24 h, can be detached from the human skin without residues of adhesive and with detachment of skin cells limited to not more than 5 % of the bonding area.

7. Polymeric, pressure-sensitive adhesive for bonding to human skin, resulting from 100 % acrylate-based monomers, namely
50 parts by weight of polyethylene glycol methacrylate 550 and
50 parts by weight of 2-ethylhexyl acrylate and
0.75 part by weight of tripropylene glycol diacrylate,
where the pressure-sensitive adhesive at a layer thickness of 250 *µ*m has a water vapour permeability, as measured
- to ASTM E 96 by the upright cup method at a temperature of 23 °C and an atmospheric humidity of 50 %, on irradiation with a UV lamp over a time of 30 s, of 1280 g/m²·24 h and
- to DIN EN 13726-2 at a temperature of 37 °C and an atmospheric humidity of 18 %, of between 1000 and 1200 g/m² over 24 h,
or
50 parts by weight of polyethylene glycol methacrylate 550 and
50 parts by weight of 2-ethylhexyl acrylate and
0.5 part by weight of tripropylene glycol diacrylate,
where the pressure-sensitive adhesive at a layer thickness of 250 *µ*m has a water vapour permeability, as measured
- to ASTM E 96 by the upright cup method at a temperature of 23 °C and an atmospheric humidity of 50 %, on irradiation with a UV lamp over a time of 30 s, of 1390 g/m²·24 h and
- to DIN EN 13726-2 at a temperature of 37 °C and an atmospheric humidity of 18 %, of between 1000 and 1200 g/m² over 24 h,
or
50 parts by weight of polyethylene glycol methacrylate 550 and
50 parts by weight of 2-ethylhexyl acrylate and
0.25 part by weight of tripropylene glycol diacrylate,
where the pressure-sensitive adhesive at a layer thickness of 250 *µ*m has a water vapour permeability, as measured
- to ASTM E 96 by the upright cup method at a temperature of 23 °C and an atmospheric humidity of 50 %, on irradiation with a UV lamp over a time of 30 s, of 1530 g/m²·24 h and
- to DIN EN 13726-2 at a temperature of 37 °C and an atmospheric humidity of 18 %, of between 1000 and 1200 g/m² over 24 h,
or
2 parts by weight of acrylic acid and
25 parts by weight of ethylhexyl acrylate and
25 parts by weight of polyethylene glycol methacrylate,
where the pressure-sensitive adhesive at a layer thickness of 250 *µ*m has a water vapour permeability, as measured
- to ASTM E 96 by the upright cup method at a temperature of 23 °C and an atmospheric humidity of 50 %, of 1070 g/m²·24 h and
- to DIN EN 13726-2 at a temperature of 37 °C and an atmospheric humidity of 18 %, of between 1000 and 1200 g/m² over 24 h.

## Revendications

1. Utilisation d'une masse adhésive polymère qui est issue à 100 % de monomères à base d'acrylate et qui présente pour une épaisseur de couche de 250 µm une transmission de vapeur d'eau d'au moins 1000 g/m² sur 24 h, mesurée
- selon la norme ASTM E 96 selon la méthode « upright cup » à une température de 23 °C et une humidité atmosphérique de 50 % et
- selon la norme DIN EN 13726-2 à une température de 37 °C et une humidité atmosphérique de 18 %,
en vue d'un collage sur la peau humaine

2. Utilisation selon la revendication 1, **caractérisée en ce que** la masse adhésive est préparée par polymérisation par UV.

3. Utilisation selon la revendication 1, **caractérisée en ce que** la masse adhésive est préparée par polymérisation thermique.

4. Utilisation selon la revendication 1, **caractérisée en ce que** la masse adhésive est préparée par polymérisation sans utilisation de solvants organiques.

5. Utilisation selon la revendication 1, **caractérisée en ce que** la masse adhésive est préparée par polymérisation à base de solvant.

6. Utilisation selon l'une quelconque des revendications précédentes, **caractérisée en ce que** la masse adhésive peut être décollée de la peau humaine même après une période de port allant jusqu'à 24 h sans résidus d'adhésif et avec un décollement maximal de cellules cutanées de la peau humaine limité à 5 % de la surface de collage.

7. Masse adhésive polymère destinée à un collage sur la peau humaine, issue à 100 % de monomères à base d'acrylate, à savoir
50 parties en poids de méthacrylate de polyéthylèneglycol 550 et
50 parties en poids d'acrylate de 2-éthylhexyle et
0,75 partie en poids de diacrylate de tripropylèneglycol,
dans laquelle la masse adhésive présente pour une épaisseur de couche de 250 µm une transmission de la vapeur d'eau mesurée
- de 1280 g/m² sur·24 h selon la norme ASTM E 96 selon la méthode « upright cup » à une température de 23 °C et une humidité atmosphérique de 50 % sous irradiation avec une lampe UV sur une période de 30 s, et
- comprise entre 1000 et 1200 g/m² sur 24 h selon la norme DIN EN 13726-2 à une température de 37 °C et une humidité atmosphérique de 18 %, ou
50 parties en poids de méthacrylate de polyéthylèneglycol 550 et
50 parties en poids d'acrylate de 2-éthylhexyle et
0,5 partie en poids de diacrylate de tripropylèneglycol,
dans laquelle la masse adhésive présente pour une épaisseur de couche de 250 µm une transmission de la vapeur d'eau mesurée
- de 1390 g/m²·sur 24 h selon la norme ASTM E 96 selon la méthode « upright cup » à une température de 23 °C et une humidité atmosphérique de 50 % sous irradiation avec une lampe UV sur une période de 30 s, et
- comprise entre 1000 et 1200 g/m² sur 24 h selon la norme DIN EN 13726-2 à une température de 37 °C et une humidité atmosphérique de 18 %, ou
50 parties en poids de méthacrylate de polyéthylèneglycol 550 et
50 parties en poids d'acrylate de 2-éthylhexyle et
0,25 parties en poids de diacrylate de tripropylèneglycol,
dans laquelle la masse adhésive présente pour une épaisseur de couche de 250 µm une transmission de la vapeur d'eau mesurée
- de 1530 g/m²·sur 24 h selon la norme ASTM E 96 selon la méthode « upright cup » à une température de 23 °C et une humidité atmosphérique de 50 % sous irradiation avec une lampe UV sur une période de 30 s, et
- comprise entre 1000 et 1200 g/m² sur 24 h selon la norme DIN EN 13726-2 à une température de 37 °C et une humidité atmosphérique de 18 %, ou
2 parties en poids d'acide acrylique et
25 parties en poids d'acrylate d'éthylhexyle et
25 parties en poids de méthacrylate de polyéthylèneglycol,
dans laquelle la masse adhésive présente pour une épaisseur de couche de 250 µm une transmission de la vapeur d'eau mesurée
- de 1070 g/m²·sur 24 h selon la norme ASTM E 96 selon la méthode « upright cup » à une température de 23 °C et une humidité atmosphérique de 50 %, et
- comprise entre 1000 et 1200 g/m² sur 24 h selon la norme DIN EN 13726-2 à une température de 37 °C et une humidité atmosphérique de 18 %.
